# EUROPÄISCHE PATENTANMELDUNG

(11) **EP 4 327 930 A1**
(43) Veröffentlichungstag der Anmeldung: **28.02.2024**
(21) Anmeldenummer: 22192298.2
(22) Anmeldetag: 26.08.2022
(51) Int. Cl.: B01J 8/04, B01J 19/24, C07C 31/04

(54) **MULTISTUFENREAKTOR ZUM DURCHFÜHREN EXOTHERMER GLEICHGEWICHTSREAKTIONEN**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Strozyk, Michael, 60439 Frankfurt am Main (DE); Castillo-Welter, Frank, 60388 Frankfurt (DE); Oelmann, Tobias, 60439 Frankfurt (DE); Schuhmann, Timm, 64625 Bensheim (DE)
(74) Vertreter: Stang, Stefan

(57) **Zusammenfassung**

Die Erfindung betrifft einen Reaktor zum Durchführen exothermer Gleichgewichtsreaktionen, insbesondere zur Herstellung von Methanol aus Synthesegas im Rahmen einer Mehrstufensynthese mit Zwischenkondensation des Reaktionsproduktes. Der erfindungsgemäße Reaktor weist einen Reaktormantel und eine innerhalb des Reaktormantels angeordnete Mehrzahl hintereinander geschalteter und in Fluidverbindung miteinander stehender Reaktorzellen auf, wobei jede der Reaktorzellen eine Reaktionsvorrichtung, eine Abkühlvorrichtung und eine Phasentrennvorrichtung als Reaktorzellenelemente umfasst. Der Reaktor weist eine Mehrzahl innerhalb des Reaktormantels und parallel zueinander angeordnete Reaktorebenen auf, wobei Reaktorzellenelemente derselben Art auf derselben Reaktorebene angeordnet sind. Die erfindungsgemäße Anordnung der Reaktorzellenelemente ermöglicht den Bau eines kompakten Reaktors und reduziert Materialspannungen innerhalb des Reaktors durch die Vermeidung großer Temperaturunterschiede innerhalb des Reaktormantels.

## Beschreibung

### Technisches Gebiet der Erfindung

Die Erfindung betrifft einen Reaktor, insbesondere einen Multistufen-Reaktor zum Durchführen exothermer Gleichgewichtsreaktionen mit Zwischenkondensation des Reaktionsprodukts. Die Erfindung betrifft ferner die Verwendung des erfindungsgemäßen Reaktors für die Herstellung von Methanol.

### Stand der Technik

Die EP 3 401 299 A1 und die EP 3 401 300 beschreiben das Prinzip eines Multistufen-Reaktors mit Zwischenkondensation, beispielsweise für die Herstellung von Methanol mit Zwischenkondensation des Rohmethanols (Methanol, Wasser und Verunreinigungen). Gemäß diesem Prinzip sind mehrere Reaktorzellen innerhalb eines gemeinsamen Mantels angeordnet, wobei jede der Reaktorzellen zumindest die eigentliche Reaktionszone, eine Abkühlzone, eine Abscheidezone sowie Mittel zum Ausleiten des abgeschiedenen Reaktionsprodukts und zum Ausleiten des nicht umgesetzten Einsatzgases umfasst.

Für die räumliche Anordnung der Reaktorzellen innerhalb des Reaktormantels wird in der EP 3 401 299 A1 ausschließlich eine Anordnung der Reaktorzellen hintereinander in vertikaler (aufrecht stehender) oder horizontaler (liegender) Weise vorgeschlagen.

Durch einfaches hintereinander Anordnen von Reaktorzellen ergeben sich jedoch unvorteilhafte Reaktormantel-Dimensionierungen.

Gemäß einem Beispiel ergibt sich für einen dreistufigen Reaktor eine Länge von etwa 7 Metern pro Stufe, davon ausgehend dass die Katalysatorbettlänge der Reaktionszone 4 Meter beträgt, der dahinter angeordnete Wärmeaustauscher eine Länge von etwa 1,5 Metern aufweist und der Kondensator und der Abscheider zusammen 1,5 Meter Länge in Anspruch nehmen. Diese Reaktorzellen aufeinander gestapelt ergeben bereits eine Gesamtlänge von 21 Metern.

Vor allem bei Reaktoren mit eher kleinen Kapazitäten mit entsprechend kleinen Reaktormantel-Durchmessern ergibt sich hier ein ungünstiges Längen-Durchmesser-Verhältnis. Es bedarf eines zusätzlichen Stahlgerüsts, um die mechanische Stabilität eines derart schlanken Apparates sicherzustellen. Auch die Handhabung des Katalysators, vor allem in den unteren Reaktorzellen, ist schwierig. Die Gasführung innerhalb der und zwischen den Reaktorzellen ist komplex und erfordert komplizierte Einbauten, Rohrführung und Strömungskanäle. Bei der Verwendung von Thermoblechen als Wärmeaustauscher-Struktur (auch bezeichnet als Kissenplatten-Wärmeübertrager oder *pillow plate heat exchanger*) müssen jeweils einzelne Thermoblechpakete mit Verteiler (*header*) und Sammler (*collector*) gefertigt und im Mantel verbaut werden, was zu höheren Produktionskosten führt. Auch die Temperaturführung ist bei einer solchen Konfiguration nicht optimal, da beispielsweise eine heiße Reaktionszone einer zweiten Reaktorzelle in direkter Nähe einer kalten Abscheidezone der vorgelagerten, ersten Reaktorzelle liegt. Dies erfordert eine entsprechend aufwändige Isolation. Auch kann die Leistung in der Abscheidezone durch Erwärmung der flüssigen Phase im Abscheider beeinträchtigt werden, was zu Einbußen bei der Produktausbeute führt. Auch die damit einhergehenden thermischen Spannungen des verwendeten Metalls erfordert eine komplexe Bauweise, die bei der Konstruktion des Apparates berücksichtigt werden muss.

Alternativ können die Reaktionszone, die Abkühlzone und die Abscheidezone horizontal in einem gemeinsamen Reaktormantel angeordnet werden. In Bezug auf die Gesamtlänge eines Apparates, entsprechend dem vorgenannten Beispiel, können hier Einsparungen bezüglich der Gesamtlänge des liegenden Apparates erzielt werden, wenn das Prozessgas von oben nach unten, senkrecht zur horizontalen Achse des liegenden Apparates, angeströmt wird. Entsprechend dem obigen Beispiel käme in diesem Fall eine Gesamtlänge von etwa 5,70 Metern zustande. Durch Teilung der Reaktionszonen kann der Manteldurchmesser verringert werden, dies führt jedoch zu einer verkomplizierten Prozessgasführung. Ein weiterer Lösungsansatz könnte in der horizontalen Anströmung des Reaktorbetts liegen. Hier besteht der Nachteil jedoch darin, dass durch die Katalysatorschrumpfung hervorgerufene Bypass-Strömung technisch schwierig in den Griff zu bekommen ist. Ferner ist über die Lebenszeit des Katalysators davon auszugehen, dass das Katalysatorbett im unteren Bereich der Schüttung sukzessive verdichtet, so dass sich über die Lebenszeit des Katalysators bei horizontaler Anströmung eine zunehmende Fehlverteilung des Prozessgases über das Katalysatorbett ergibt. Wie im obigen Beispiel mit vertikaler Anordnung ist auch bei horizontaler, räumlich aneinander gereihter Anordnung der Reaktorzellen die Temperaturführung mit Nachteilen behaftet und die Prozessgasführung kompliziert.

US 2018/0221842 A1 betrifft einen mehrstufigen Reaktor und ein System zur Herstellung von Methanol aus Synthesegas. Der Reaktor enthält einen Rohrbündelreaktor, der an seinem oberen und unteren Ende in mehrere vertikale, isolierte Kompartimente unterteilt ist. Die zugehörigen Kompartimente und Rohre bilden eine Stufe des Reaktors. Das rohe Synthesegas wird der ersten Stufe zugeführt, und das nicht umgesetzte Synthesegas aus der ersten Stufe wird der zweiten, nachfolgenden Stufe zugeführt. Zwischen den einzelnen Stufen wird das Produkt, Methanol und Wasser, aus dem Reaktionsgemisch entfernt, bevor das nicht umgesetzte Synthesegas in die nachfolgende Stufe geleitet wird. Die Wärmeaustauscher und Abscheider sind außerhalb des Reaktormantels angeordnet, was in einer komplizierten Anordnung von Leitungen und einer komplexen Prozessführung führt.

### Beschreibung der Erfindung

Eine Aufgabe der vorliegenden Erfindung besteht darin, die vorgenannten Nachteile des Standes der Technik zumindest teilweise zu überwinden.

Insbesondere besteht eine Aufgabe der vorliegenden Erfindung darin, einen Reaktor bereitzustellen, der die Anordnung einer Mehrzahl von Reaktionsvorrichtungen, Abkühlvorrichtungen und Phasentrennvorrichtungen innerhalb eines kompakten Gehäuses ermöglicht.

Eine weitere Aufgabe der vorliegenden Erfindung besteht darin, einen Reaktor bereitzustellen, der die Anordnung einer Mehrzahl von Reaktionsvorrichtungen, Abkühlvorrichtungen und Phasentrennvorrichtungen innerhalb eines gemeinsamen Reaktormantels ermöglicht.

Eine weitere Aufgabe der vorliegenden Erfindung besteht darin, einen Reaktor bereitzustellen, welcher bei Vorhandensein einer Mehrzahl von Reaktionsvorrichtungen und Phasentrennvorrichtungen die Anordnung einer Reaktionsvorrichtung und einer Phasentrennvorrichtung direkt nebeneinander vermeidet.

Eine weitere Aufgabe der vorliegenden Erfindung besteht darin, einen Reaktor bereitzustellen, welcher bei Vorhandensein einer Mehrzahl von Reaktionsvorrichtungen, Abkühlvorrichtungen und Phasentrennvorrichtungen ein vergleichmäßigtes Temperaturprofil über die Gesamtlänge des Reaktors ermöglicht, so dass Materialspannungen vermieden werden und kostengünstigere Materialien bezüglich der Reaktorbauteile Verwendung finden können.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie.

Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

Gemäß einem ersten Aspekt der Erfindung wird ein Reaktor zum Durchführen exothermer Gleichgewichtsreaktionen vorgeschlagen, in dem ein Einsatzgasgemisch und/oder ein Restgasgemisch an einem festen Katalysator zumindest teilweise umsetzbar ist/sind, wobei ein Produktgasgemisch und ein Restgasgemisch erhältlich sind, wobei das Produktgasgemisch ein bei Reaktionsdruck und unterhalb der Reaktionstemperatur kondensierbares, flüssiges Reaktionsprodukt enthält, aufweisend einen Reaktormantel und eine innerhalb des Reaktormantels angeordnete Mehrzahl hintereinander geschalteter und in Fluidverbindung miteinander stehender Reaktorzellen, wobei
jede Reaktorzelle die folgenden, hintereinander geschalteten und in Fluidverbindung miteinander stehenden Reaktorzellenelemente umfasst:
- Eine Reaktionsvorrichtung, konfiguriert zum Umsetzen des Einsatzgasgemischs und/oder des Restgasgemischs zum Produktgasgemisch, aufweisend den festen Katalysator und eine mit dem festen Katalysator in Wärmeaustauschbeziehung stehende und von einem Kühlmedium durchströmbare Kühlvorrichtung;
- Eine Abkühlvorrichtung, konfiguriert zum Abkühlen des aus der Reaktionsvorrichtung austretenden Produktgasgemischs;
- Eine Phasentrennvorrichtung, konfiguriert zum Kondensieren und Abscheiden des flüssigen Reaktionsprodukts aus dem abgekühlten Produktgasgemisch, wobei die kondensierte, flüssige Phase das Reaktionsprodukt und die gasförmige Phase das Restgasgemisch enthält;

und wobei jede Reaktorzelle
   - Mittel zum Ausleiten des Reaktionsprodukts aus der Reaktorzelle umfasst, und
   - Mittel zum Ausleiten des Restgasgemischs aus der Reaktorzelle umfasst, und
   - Mittel zum Zuführen des Restgasgemischs zu einer, soweit vorhanden, stromabwärts angeordneten Reaktorzelle umfasst;
und wobei der Reaktor
   - eine Mehrzahl innerhalb des Reaktormantels und parallel zueinander angeordnete Reaktorebenen aufweist, wobei Reaktorzellenelemente derselben Art auf derselben Reaktorebene angeordnet sind.

Der erfindungsgemäße Reaktor ist zum Durchführen exothermer Gleichgewichtsreaktionen konfiguriert, insbesondere zum Durchführen der Synthese von Methanol im Industriemaßstab aus Synthesegas. Bei dem Einsatzgasgemisch handelt es sich insbesondere um ein Synthesegasgemisch, wobei das Synthesegasgemisch zumindest Wasserstoff als ein erstes Eduktgas aufweist, sowie zumindest Kohlenmonoxid oder Kohlendioxid als zweites Eduktgas aufweist. Insbesondere weist das Einsatzgasgemisch Wasserstoff als erstes Eduktgas, Kohlenmonoxid als zweites Eduktgas, sowie Kohlendioxid als drittes Eduktgas auf.

Zumindest ein Teil der Reaktorzellenelemente sind mechanisch mit dem Reaktormantel verbunden. Dabei ist es nicht zwingend erforderlich, dass jedes der Reaktorzellenelemente mechanisch mit dem Reaktormantel verbunden ist. Gemäß einer Ausführungsform sind Reaktorzellenelemente untereinander durch mechanische Verbindungselemente miteinander verbunden, so dass nicht für jedes der Reaktorzellenelemente eine mechanische Verbindung zum Reaktormantel erforderlich ist. Bei der mechanischen Verbindung kann es sich um eine mechanische Stützstruktur, beispielsweise in Form einer Aufhängung oder Abstützung, handeln.

Die Bildung des Produktgasgemischs erfolgt bei erhöhter Temperatur (Reaktionstemperatur) und bei erhöhtem Druck. Handelt es sich bei dem Reaktionsprodukt beispielsweise um Methanol und Wasser, so erfolgt die Bildung des Produktgasgemischs insbesondere bei Temperaturen von 180 °C bis 250 °C und bei Drücken von 30 bar bis 120 bar, insbesondere bei 40 bar bis 90 bar. Bezüglich der Temperatur ist dabei der Arbeitstemperaturbereich des verwendeten Katalysators zu beachten, welcher nicht unterschritten oder überschritten werden sollte. Für die Methanolsynthese kommen in der Regel dem Fachmann bekannte feste Katalysatoren auf Kupferbasis zum Einsatz.

Das Produktgasgemisch enthält insbesondere Methanol und Wasser sowie unvermeidbare Nebenprodukte wie Ether, Ester, höhere Alkohole und Ketone, wobei das Gemisch aus Methanol, Wasser und Nebenprodukten auch als Rohmethanol bezeichnet wird. Zumindest Methanol und Wasser sind in diesem Fall die bei Reaktionsdruck und unterhalb der jeweiligen Reaktionstemperatur kondensierbaren, flüssigen Reaktionsprodukte. Da es sich bei der Bildung von Methanol und Wasser aus Synthesegas um eine Gleichgewichtsreaktion handelt, bei der sich ein thermodynamisches Gleichgewicht einstellt, wird ein Anteil des Einsatzgasgemisches, insbesondere Synthesegasgemisch, nicht zu Methanol und Wasser umgesetzt. Dieser nicht umgesetzte Anteil bildet das Restgasgemisch.

Der erfindungsgemäße Reaktor weist einen Reaktormantel und eine innerhalb des Reaktormantels angeordnete Mehrzahl hintereinander geschalteter und in Fluidverbindung miteinander stehende Reaktorzellen auf. "Mehrzahl" bedeutet in diesem Zusammenhang, dass der Reaktor zumindest zwei Reaktorzellen umfasst. Jede Reaktorzelle bildet eine Reaktionsstufe einer Mehrstufensynthese. Mehrstufensynthese bedeutet, dass in jeder Stufe der Mehrstufensynthese die Bildung von Produktgasgemisch aus Einsatzgasgemisch und/oder Restgasgemisch am festen Katalysator und die zumindest teilweise Abtrennung des gewünschten flüssigen Reaktionsprodukts aus dem erhaltenen Produktgasgemisch erfolgt. Die Reaktorzellen sind dabei hintereinander angeordnet, das heißt die zweite Reaktorzelle ist in Gasflussrichtung stromabwärts zur ersten Reaktorzelle angeordnet, die dritte Reaktorzelle, soweit vorhanden, in Gasflussrichtung stromabwärts zur zweiten Reaktorzelle und so weiter. Die Reaktorzellen stehen dabei untereinander in Fluidverbindung. Das bedeutet insbesondere, dass ein Gasgemisch, insbesondere Restgasgemisch, welches aus einer Reaktorzelle ausgeschleust wird, anschließend in die jeweilige in Gasflussrichtung stromabwärts angeordnete Reaktorzelle eingeschleust wird.

Das Produktgasgemisch wird in der ersten Reaktorzelle soweit abgekühlt, dass dieses zumindest teilweise kondensiert und so das flüssige Reaktionsprodukt aus der ersten Reaktorzelle ausgeschleust werden kann. Das Restgasgemisch wird zumindest teilweise in die zur ersten Reaktorzelle in Gasflussrichtung stromabwärts angeordnete Reaktorzelle eingeschleust, damit in der zweiten Reaktorzelle erneut Produktgasgemisch aus Restgasgemisch am festen Katalysator der zweiten Reaktorzelle gebildet werden kann. Gemäß einer Ausführungsform kann dem Restgasgemisch, welches in die zweite Reaktionszelle eingeschleust wird, auch ein Anteil Einsatzgasgemisch ("Frischgas") zugemischt werden. Produktgasgemisch der zweiten Reaktorzelle wird erneut soweit abgekühlt, dass aus diesem das Reaktionsprodukt zumindest teilweise kondensiert und in flüssiger Form aus der zweiten Reaktorzelle ausgeschleust werden kann. Ist eine dritte Reaktorzelle vorhanden, so wird das in der zweiten Reaktorzelle verbliebene Restgasgemisch sowie optional ein Anteil Einsatzgasgemisch in die dritte Reaktorzelle eingeschleust und so weiter.

Jede der Mehrzahl an Reaktorzellen umfasst eine Reaktionsvorrichtung, eine Abkühlvorrichtung und eine Phasentrennvorrichtung. Bei der Reaktionsvorrichtung, der Abkühlvorrichtung, und der Phasentrennvorrichtung handelt es sich im Sinne des erfindungsgemäßen Reaktors um unterschiedliche Arten von Reaktorzellenelementen, welche jeweils innerhalb einer Reaktorzelle angeordnet sind. Jede Reaktorzelle umfasst dabei zumindest eine Reaktionsvorrichtung, eine Abkühlvorrichtung und eine Phasentrennvorrichtung.

Bei der Reaktionsvorrichtung handelt es sich um eine Vorrichtung, welche für die Bildung des Produktgasgemischs aus dem Einsatzgasgemisch und/oder Restgasgemisch am festen Katalysator konfiguriert ist. Die Reaktionsvorrichtung weist dabei eine mit dem festen Katalysator in Wärmeaustauschbeziehung stehende Kühlvorrichtung auf. Die Kühlvorrichtung dient insbesondere der Temperaturkontrolle der exothermen Gleichgewichtsreaktion, das heißt der Abführung von Reaktionswärme. Dabei ist die Kühlvorrichtung insbesondere so konfiguriert, dass eine bestimmte festgelegte Maximaltemperatur im Katalysatorbett des festen Katalysators nicht überschritten wird. Gemäß einer Ausführungsform handelt es sich bei dem Kühlmedium um siedendes Kesselspeisewasser, wobei die Kühlung in der Reaktionsvorrichtung nach dem Prinzip der Siedekühlung erfolgt. Dabei wird aus dem siedenden Kesselspeisewasser Dampf gebildet, welcher zum Zwecke der Energierückgewinnung beispielsweise als Exportdampf genutzt werden kann. Bei dem festen Katalysator kann es sich um ein Katalysatorbett auf der Basis von Katalysatorpartikeln (Pellets) oder um einen strukturierten Katalysator, beispielsweise mit monolithischer Struktur, handeln.

Jede der Reaktorzellen umfasst darüber hinausgehend zumindest eine Abkühlvorrichtung. Die Abkühlvorrichtung dient dem Abkühlen des aus der Reaktionsvorrichtung austretenden Produktgasgemischs. Dabei wird das Produktgasgemisch in der Abkühlvorrichtung vorzugsweise nur soweit abgekühlt, dass noch keine Kondensation des flüssigen Reaktionsprodukts erfolgt, das heißt das Reaktionsprodukt verbleibt innerhalb der Abkühlvorrichtung in der Gasphase. Bei dem Reaktionsprodukt handelt es sich insbesondere um ein Gemisch aus Methanol und Wasser, sowie gegebenenfalls weitere kondensierbare und/oder nicht kondensierbare (unerwünschte) Nebenprodukte. Innerhalb einer Reaktorzelle ist die Abkühlvorrichtung in Gasflussrichtung stromabwärts zur Reaktionsvorrichtung der gleichen Reaktorzelle angeordnet. Vorzugsweise weist die Abkühlvorrichtung eine Kühlvorrichtung auf, welche in Wärmeaustauschbeziehung mit dem aus der Reaktionsvorrichtung austretenden Produktgasgemisch steht. Insbesondere handelt es sich bei der Kühlvorrichtung um einen indirekten Wärmeaustauscher, wobei als Kühlmedium ein Gas oder eine Flüssigkeit in Frage kommen. Vorzugsweise handelt es sich bei dem Kühlmedium um das Einsatzgasgemisch oder Restgasgemisch. Handelt es sich dabei um das Einsatzgasgemisch, so wird dieses vorzugsweise der Abkühlvorrichtung der ersten Reaktorzelle als Kühlmedium zugeführt. Handelt es sich dabei um das Restgasgemisch, so wird dieses vorzugsweise einer Abkühlvorrichtung einer der zur ersten Reaktorzelle in Gasflussrichtung stromabwärts angeordneten Reaktorzelle zugeführt, also der zweiten und/oder dritten Reaktorzelle und so weiter. Insbesondere wird dieses Restgasgemisch aus der Phasentrennvorrichtung der der jeweiligen Reaktorzelle vorgeschalteten Reaktorzelle abgezogen, also aus der Phasentrennvorrichtung der ersten Reaktorzelle, um es der Abkühlvorrichtung der zweiten Reaktorzelle zuzuführen, und/oder aus der Phasentrennvorrichtung der zweiten Reaktorzelle, um es der Abkühlvorrichtung der dritten Reaktorzelle zuzuführen und so weiter.

Jede der Reaktorzellen umfasst darüber hinausgehend zumindest eine Phasentrennvorrichtung. Die Phasentrennvorrichtung dient dem weiteren Abkühlen des in der Abkühlvorrichtung bereits vorgekühlten Produktgasgemischs, so dass das Reaktionsprodukt kondensiert und in flüssiger Form erhalten wird. Die Phasentrennvorrichtung ist somit so konfiguriert, dass eine kondensierte, flüssige Phase und eine gasförmige Phase erhalten werden. Des Weiteren dient die Phasentrennvorrichtung der Abtrennung der flüssigen Phase von der gasförmigen Phase, insbesondere des flüssigen Reaktionsprodukts von den weiteren Bestandteilen, also nicht verflüssigten, das heißt in der Phasentrennvorrichtung nicht kondensierten oder nicht kondensierbaren Bestandteilen. Handelt es sich bei dem Einsatzgasgemisch um ein Synthesegasgemisch und bei dem Zielprodukt um Methanol, so enthält die flüssige Phase als kondensiertes, flüssiges Reaktionsprodukt Methanol und Wasser, und unter den jeweiligen Bedingungen kondensierbare (unerwünschte) Nebenprodukte. Die gasförmige Phase enthält das Restgasgemisch, also nicht umgesetztes Synthesegas sowie unter den jeweiligen Bedingungen nicht kondensierbare Nebenprodukte. Nicht kondensierbare Nebenprodukte schließen auch Bestandteile ein, welche bereits im Einsatzgasgemisch enthalten sind und unter den Bedingungen der Methanolsynthese inert sind, das heißt weder zum Zielprodukt Methanol noch zu einem Nebenprodukt umgesetzt werden. Beispiele für diese Art von Nebenprodukten sind Methan und Stickstoff.

Innerhalb einer Reaktorzelle ist die Phasentrennvorrichtung in Gasflussrichtung stromabwärts zur Abkühlvorrichtung der gleichen Reaktorzelle angeordnet. Vorzugsweise weist die Phasentrennvorrichtung eine Kühlvorrichtung auf, welche in Wärmeaustauschbeziehung mit dem aus der Abkühlvorrichtung austretenden Produktgasgemisch steht. Insbesondere handelt es sich bei der Kühlvorrichtung um einen indirekten Wärmeaustauscher, wobei als Kühlmedium vorzugsweise eine Flüssigkeit, insbesondere Kühlwasser in Frage kommt. Die Phasentrennvorrichtung weist darüber hinausgehend weiterhin vorzugsweise eine Vorrichtung zur Phasentrennung auf, insbesondere einen Gas-Flüssigkeits-Abscheider.

Der erfindungsgemäße Reaktor weist eine Mehrzahl von Reaktorzellen auf, wobei diese Reaktorzellen hintereinander geschaltet sind. Das heißt, in Gasflussrichtung folgt auf die erste Reaktorzelle eine zweite Reaktorzelle, auf die zweite Reaktorzelle optional eine dritte Reaktorzelle und so weiter. Weist der Reaktor eine Anzahl N Reaktorzellen auf, so die erste Reaktorzelle die [N-(N-1)]-te Reaktorzelle, die zweite Reaktorzelle die [N-(N-2)]-te Reaktorzelle und so weiter, und die letzte Reaktorzelle ist die [N-(N-N)]-te oder N-te Reaktorzelle. Jede der Reaktorzellen weist zumindest eine Reaktionsvorrichtung, eine Abkühlvorrichtung, sowie eine Phasentrennvorrichtung auf, wobei die Abkühlvorrichtung jeweils in Gasflussrichtung stromabwärts zur Reaktionsvorrichtung und die Phasentrennvorrichtung jeweils stromabwärts zur Abkühlvorrichtung einer Reaktorzelle angeordnet ist. Aufgrund der Anordnung der Reaktorzellen hintereinander ist die Reaktionsvorrichtung einer zweiten Reaktorzelle dabei in Gasflussrichtung stromabwärts zur Phasentrennvorrichtung einer ersten Reaktorzelle angeordnet. Die Reaktionsvorrichtung einer dritten Reaktorzelle ist stromabwärts zur Phasentrennvorrichtung einer zweiten Reaktorzelle angeordnet und so weiter. Enthält ein erfindungsgemäßer Reaktor beispielsweise insgesamt drei Reaktorzellen, so ergibt sich für die Reihenfolge in Gasflussrichtung folgende Anordnung, angenommen dass jede Reaktorzelle eine Reaktionsvorrichtung, eine Abkühlvorrichtung und eine Phasentrennvorrichtung aufweist und jede dieser Reaktorzellenelemente als Index (in Klammern) die Nummer der Reaktorzelle trägt:
Reaktionsvorrichtung (1) → Abkühlvorrichtung (1) → Phasentrennvorrichtung (1) → Reaktionsvorrichtung (2) → Abkühlvorrichtung (2) → Phasentrennvorrichtung (2) → Reaktionsvorrichtung (3) → Abkühlvorrichtung (3) → Phasentrennvorrichtung (3).

Weist der Reaktor eine Anzahl N Reaktorzellen auf, so ergibt sich im Allgemeinen folgende Reihenfolge, angenommen dass jede Reaktorzelle eine Reaktionsvorrichtung, eine Abkühlvorrichtung und eine Phasentrennvorrichtung aufweist:
Reaktionsvorrichtung ([N-(N-1)]) → Abkühlvorrichtung ([N-(N-1)]) → Phasentrennvorrichtung ([N-(N-1)]) → Reaktionsvorrichtung ([N-(N-2)]) → Abkühlvorrichtung ([N-(N-2)]) → Phasentrennvorrichtung ([N-(N-2)]) → [...] → Reaktionsvorrichtung (([N-(N-N)]) → Abkühlvorrichtung ([N-(N-N)]) → Phasentrennvorrichtung ([N-(N-N)]).

Jede der Reaktorzellen umfasst Mittel zum Ausleiten des Reaktionsprodukts aus der jeweiligen Reaktorzelle. Insbesondere umfasst der erfindungsgemäße Reaktor Mittel zum Ausleiten des flüssigen Reaktionsprodukts aus der Phasentrennvorrichtung der jeweiligen Reaktorzelle. Das aus jeder der Reaktorzellen ausgeleitete flüssige Reaktionsprodukt, bei dem es sich insbesondere um Rohmethanol handelt, wird anschließend einer weiteren Aufarbeitung, zum Beispiel Rektifikation, zugeführt. Gemäß einer Ausführungsform werden die Ströme an flüssigem Reaktionsprodukt, welche aus jeder der Reaktorzellen ausgeleitet werden, zusammengeführt und gemeinsam einer weiteren Aufarbeitung unterzogen.

Jede der Reaktorzellen umfasst ferner Mittel zum Ausleiten des Restgasgemischs aus der jeweiligen Reaktorzelle. Insbesondere umfasst der erfindungsgemäße Reaktor Mittel zum Ausleiten des Restgasgemischs aus der Phasentrennvorrichtung der jeweiligen Reaktorzelle. Das aus jeder der Reaktorzellen ausgeleitete Restgasgemisch enthält im Falle der Methanolsynthese insbesondere nicht umgesetztes Synthesegasgemisch, somit nicht umgesetzten Wasserstoff sowie Kohlenmonoxid und/oder Kohlendioxid. Ferner enthält das Restgasgemisch nicht kondensierbare Nebenprodukte, beispielsweise Methan und Stickstoff.

Ferner umfasst jede der Reaktorzellen Mittel zum Zuführen des Restgasgemischs zu einer stromabwärts angeordneten Reaktorzelle, soweit eine stromabwärts angeordnete Reaktorzelle vorhanden ist. "Stromabwärts angeordnet" bedeutet in diesem Zusammenhang, dass die Reaktorzelle in Gasflussrichtung stromabwärts zur vorherigen Reaktorzelle angeordnet ist. Insbesondere umfasst jede der Reaktorzellen Mittel zum Zuführen des Restgasgemischs zu einer stromabwärts angeordneten Reaktionsvorrichtung, soweit eine stromabwärts angeordnete Reaktionsvorrichtung vorhanden ist. Handelt es sich bei der Reaktorzelle um die letzte Reaktorzelle der N Reaktorzellen, also die N-te Reaktorzelle, ist keine stromabwärts angeordnete Reaktorzelle vorhanden. An dieser Stelle nach wie vor nicht umgesetztes Restgasgemisch kann optional wieder zur ersten Reaktorzelle zurückgeführt werden. Gemäß dieser Ausführungsform weist die letzte oder N-te Reaktorzelle Mittel zum Zuführen des Restgasgemischs zur ersten oder [N-(N-1)]-ten Reaktorzelle, insbesondere zur Reaktionsvorrichtung der ersten oder [N-(N-1)]-ten Reaktorzelle auf. Gemäß einer Ausführungsform wird aus dem aus der letzten Reaktorzelle ausgeleiteten Restgas ein Purgegas abgezogen, um die Anreicherung von inerten Komponenten im Reaktor zu vermeiden.

Erfindungsgemäß weist der Reaktor eine Mehrzahl innerhalb des Reaktormantels und parallel zueinander angeordnete Reaktorebenen auf, wobei Reaktorzellenelemente derselben Art auf derselben Reaktorebene angeordnet sind. Insbesondere ist eine Mehrzahl an Reaktorzellenelementen derselben Art auf derselben, also auf der gleichen Reaktorebene, angeordnet. Beispielsweise ist auf ein und derselben Reaktorebene eine Mehrzahl an Reaktionsvorrichtungen angeordnet, auf einer weiteren Reaktorebene eine Mehrzahl an Abkühlvorrichtungen angeordnet, und auf einer weiteren Reaktorebene ist eine Mehrzahl an Phasentrennvorrichtungen angeordnet. Unter einer Ebene ist dabei insbesondere eine Fläche zu verstehen, die sich über eine Querschnittsfläche des erfindungsgemäßen Reaktors erstreckt und senkrecht zu einer Hauptachse des Reaktors angeordnet ist. Der Reaktor weist eine Mehrzahl solcher Reaktorebenen auf, die parallel zueinander angeordnet sind. Erstreckt sich der erfindungsgemäße Reaktor beispielsweise in vertikaler Hauptrichtung und weist somit eine vertikale Hauptachse auf, so erstrecken sich die Reaktorebenen senkrecht zur Hauptachse des Reaktors in horizontaler Richtung und sind parallel zueinander übereinander angeordnet. Dabei ist unter einer Reaktorebene nicht zwangsläufig etwas dingliches, beispielsweise eine Platte zu verstehen. Unter einer "Reaktorebene" wird im Sinne der vorliegenden Erfindung auch, ohne darauf beschränkt zu sein, eine virtuelle Reaktorebene verstanden, was in Konsequenz bedeutet dass die Mehrzahl der jeweiligen Reaktorzellenelemente einer Art gemäß dem obigen Beispiel im Wesentlichen auf gleicher Höhe, also auf einer Ebene, angeordnet sind. Unter einer Reaktorebene kann erfindungsgemäß somit sowohl eine dingliche als auch eine virtuelle Reaktorebene verstanden werden.

Durch die erfindungsgemäße Anordnung ergibt sich beispielsweise der Vorteil, dass die Reaktionsvorrichtungen grundsätzlich so beabstandet von den Phasentrennvorrichtungen angeordnet sind, dass ein Aufheizen der Medien in den Phasentrennvorrichtungen durch die Reaktionsvorrichtungen grundsätzlich vermieden wird. Da eine Abkühlvorrichtung in Gasflussrichtung stromabwärts zu einer Reaktionsvorrichtung angeordnet ist, und eine Phasentrennvorrichtung stromabwärts zu einer Abkühlvorrichtung angeordnet ist, würden sich in einem solchen Fall auf einer ersten Ebene die Reaktionsvorrichtungen befinden, auf einer zweiten Ebene die Abkühlvorrichtungen und auf einer dritten Ebene die Phasentrennvorrichtungen. Dadurch ist eine räumliche Trennung der Reaktionsvorrichtungen und der Phasentrennvorrichtungen sichergestellt.

Durch die erfindungsgemäße Anordnung der Reaktorzellenelemente ergibt sich ferner der Vorteil einer kurzen Druckmantel Länge im Vergleich zu einer Anordnung, in welcher alle Reaktorzellenelemente entlang einer einzigen Hauptachse angeordnet wären, wobei es keine Rolle spielt ob der Reaktor dann liegend oder stehend angeordnet wäre. Gemäß der Erfindung ist das Verhältnis der Länge zum Durchmesser des Reaktors in jedem Fall kleiner und damit günstiger dimensioniert.

Weiterhin ermöglicht die erfindungsgemäße Anordnung, dass die unterschiedlichen Reaktorzellenelemente in einer Reihenfolge von "heiß nach kalt" angeordnet werden können, wobei die auf einer Reaktorebene herrschenden Temperaturen in der Reaktorzelle im Wesentlichen gleich wären. Dadurch treten wenige bis keine Wärmespannungen in radialer Richtung des Reaktors auf. Beispielsweise kann von einer zur jeweils nächsten Reaktorebene eine Anordnung zunächst der Reaktionsvorrichtungen (heiß), dann der Abkühlvorrichtungen (kälter), und dann der Phasentrennvorrichtungen (noch kälter) erfolgen. So treten auch entlang der Hauptachse des Reaktors, beispielsweise in vertikaler Richtung, bei entsprechender Anordnung der Reaktorzellenelemente keine extremen Temperatursprünge auf, wie dies beispielsweise bei einer Nebeneinanderanordnung einer Reaktionsvorrichtung und einer Phasentrennvorrichtung der Fall wäre. Eine solche nachteilbehaftete Anordnung kann durch die erfindungsgemäße Anordnung der Reaktorzellenelemente vermieden werden.

Die erfindungsgemäße Anordnung ermöglicht ferner einen modularen Aufbau des Reaktors, welcher eine Erweiterung um weitere Reaktorzellen zu einem späteren Zeitpunkt erlaubt.

Eine bevorzugte Ausführungsform des erfindungsgemäßen Reaktors ist dadurch gekennzeichnet, dass auf jeder der Reaktorebenen ausschließlich eine Art eines Reaktorzellenelements angeordnet ist.

Beispielsweise sind auf einer ersten Reaktorebene nur eine Mehrzahl an Reaktionsvorrichtungen angeordnet, auf einer zweiten Reaktorebene nur eine Mehrzahl an Abkühlvorrichtungen und auf einer dritten Reaktorebene nur eine Mehrzahl an Phasentrennvorrichtungen. Dadurch wird eine maximal kompakte Bauweise des Reaktors ermöglicht, unabhängig davon wie viele Reaktorzellen und damit einhergehend wie viele Reaktorzellenelemente der Reaktor umfasst.

Eine bevorzugte Ausführungsform des erfindungsgemäßen Reaktors ist dadurch gekennzeichnet, dass die Zahl der vorhandenen Reaktorebenen mindestens der Zahl der vorhandenen Arten von Reaktorzellenelementen entspricht.

Weist der erfindungsgemäße Reaktor beispielsweise drei verschiedene Arten an Reaktorzellenelementen auf, insbesondere eine Reaktionsvorrichtung, eine Abkühlvorrichtung und eine Phasentrennvorrichtung, so entspricht die Zahl der vorhandenen Reaktorebenen mindestens der Zahl der vorhandenen Arten von Reaktorzellenelemente, also der Zahl drei oder mehr, vorzugsweise drei.

Vorzugsweise ist der erfindungsgemäße Reaktor daher dadurch gekennzeichnet, dass die Zahl der vorhandenen Reaktorebenen der Zahl der vorhandenen Arten von Reaktorzellenelementen entspricht.

Eine bevorzugte Ausführungsform des erfindungsgemäßen Reaktors ist dadurch gekennzeichnet, dass die Reaktorzellen vertikal gegenüber der durch die Schwerkraft vermittelten Senkrechten angeordnet sind, so dass Ströme innerhalb der und zwischen den Reaktorzellenelementen von unten nach oben und von oben nach unten führbar sind.

Die vertikale Anordnung der Reaktorzellen innerhalb des Reaktormantels führt dazu, dass sich der gesamte Reaktor aufgrund der hintereinander angeordneten unterschiedlichen Reaktorzellenelemente einer Reaktorzelle als Hauptausdehnung in vertikaler Richtung erstreckt. Gleichzeitig erstreckt sich der Reaktor weniger in horizontaler Ausdehnung. Dadurch wird für den gesamten Reaktor auch bei Vorhandensein einer Vielzahl von Reaktorzellen eine vergleichsweise geringe Aufstellfläche benötigt. Gleichzeitig ist der gesamte Reaktor auch nicht übermäßig hoch, da alle Reaktorzellenelemente der gleichen Art jeweils auf einer gemeinsamen Reaktorebene angeordnet sind.

Eine bevorzugte Ausführungsform des erfindungsgemäßen Reaktors ist dadurch gekennzeichnet, dass die Reaktorzellenelemente innerhalb des Reaktormantels gemäß einer zweidimensionalen Matrix mit einer Anzahl y Spalten und einer Anzahl x Zeilen angeordnet sind, wobei die Anzahl y Spalten der Anzahl der Reaktorzellen entspricht, und die Anzahl x Zeilen der Anzahl der unterschiedlichen Arten von Reaktorzellenelementen entspricht, wobei eine Spalte der Matrix eine Reaktorzelle mit jeweils einer der unterschiedlichen Arten an Reaktorzellenelementen umfasst und eine Zeile der Matrix eine Reaktorebene aufweisend eine Reihe aus Reaktorzellenelementen derselben Art umfasst.

Durch eine Anordnung der Reaktorzellenelemente im Sinne einer zweidimensionalen Matrix wird eine maximale Minimierung des durch den Reaktor beanspruchten Volumens erreicht.

Eine bevorzugte Ausführungsform des erfindungsgemäßen Reaktors ist dadurch gekennzeichnet, dass die Reaktorebenen parallel zueinander und hintereinander von oben nach unten angeordnet sind.

Insbesondere erstrecken sich die Reaktorebenen gemäß dieser Ausführungsform in horizontaler Richtung, so dass die Anordnung der Reaktorebenen als Stapel von horizontal angeordneten Reaktorebenen zu verstehen ist, wobei die Abstände zwischen den einzelnen Reaktorebenen im Wesentlichen der durch das jeweilige Reaktorzellenelement beanspruchten Höhe entspricht.

Eine bevorzugte Ausführungsform des erfindungsgemäßen Reaktors ist dadurch gekennzeichnet, dass der Reaktor ein erstes Ende und ein zweites Ende aufweist, wobei auf den Reaktorebenen vom ersten Ende aus in Richtung des zweiten Endes Reaktorzellenelemente räumlich gemäß folgender Reihenfolge angeordnet sind: Reaktionsvorrichtung, Abkühlvorrichtung, Phasentrennvorrichtung.

Zur maximalen Vereinfachung der Stromführung und zur maximalen Beabstandung der Phasentrennvorrichtungen von den Reaktionsvorrichtungen sind die einzelnen Reaktorzellenelemente innerhalb einer Reaktorzelle gemäß der obigen Reihenfolge angeordnet. Ein solcher Reaktor umfasst somit insgesamt drei Reaktorebenen, wobei die Reihenfolge der Anordnung der Reaktorzellenelemente je Reaktorzelle vom ersten Ende hin zum zweiten Ende wie folgt ist: Reaktionsvorrichtung, Abkühlvorrichtung, Phasentrennvorrichtung. Die Begriffe "erstes Ende" und "zweites Ende" sind dabei in Bezug auf die räumliche Anordnung der Reaktorzellenelemente innerhalb des Reaktormantels zu verstehen, nicht auf die Strömungsführung innerhalb des Reaktors.

Eine bevorzugte Ausführungsform des erfindungsgemäßen Reaktors ist dadurch gekennzeichnet, dass jede Phasentrennvorrichtung eine Kondensationsvorrichtung und eine Abscheidevorrichtung umfasst, wobei die Kondensationsvorrichtungen und die Abscheidevorrichtungen jeweils auf unterschiedlichen Reaktorebenen angeordnet sind.

Gemäß dieser Ausführungsform wird die Phasentrennvorrichtung als zwei voneinander getrennten Vorrichtungen ausgestaltet, welche untereinander in Fluidverbindung stehen. Insbesondere ist die Kondensationsvorrichtung als Kondensator ausgestaltet, welcher beispielsweise mit Kühlwasser als Kühlmedium betrieben wird. Ferner ist die Abscheidevorrichtung insbesondere als Gas-Flüssigkeits-Abscheider ausgestaltet, in welchem die Trennung des flüssigen Reaktionsprodukts von dem gasförmigen Restgasgemisch erfolgt.

In diesem Zusammenhang ist eine bevorzugte Ausführungsform des erfindungsgemäßen Reaktors dadurch gekennzeichnet, dass der Reaktor ein erstes Ende und ein zweites Ende aufweist, wobei auf den Reaktorebenen vom ersten Ende aus in Richtung des zweiten Endes Kondensationsvorrichtungen und Abscheidevorrichtungen räumlich gemäß folgender Reihenfolge angeordnet sind: Kondensationsvorrichtungen, Abscheidevorrichtungen.

Sind die Phasentrennvorrichtungen als getrennte Kondensationsvorrichtungen und Abscheidevorrichtungen ausgestaltet, so umfasst der Reaktor somit insgesamt vier Reaktorebenen, wobei die Reihenfolge der Anordnung der Reaktorzellenelemente je Reaktorzelle vom ersten Ende hin zum zweiten Ende wie folgt ist: Reaktionsvorrichtung, Abkühlvorrichtung, Kondensationsvorrichtung, Abscheidevorrichtung.

Eine bevorzugte Ausführungsform des erfindungsgemäßen Reaktors ist dadurch gekennzeichnet, dass der gesamte Reaktor vertikal gegenüber der durch die Schwerkraft vermittelten Senkrechten angeordnet ist und sich das erste Ende oben befindet und das zweite Ende unten befindet.

Eine bevorzugte Ausführungsform des erfindungsgemäßen Reaktors ist dadurch gekennzeichnet, dass die Reaktionsvorrichtungen auf einer obersten Reaktorebene angeordnet sind, die Phasentrennvorrichtungen auf einer untersten Reaktorebene angeordnet sind, und die Abkühlvorrichtungen auf einer Reaktorebene zwischen der obersten und untersten Reaktorebene angeordnet sind.

Sind die Phasentrennvorrichtungen als getrennte Kondensationsvorrichtungen und Abscheidevorrichtungen ausgestaltet, so sind die Reaktionsvorrichtungen auf einer obersten Reaktorebene angeordnet, die Abscheidevorrichtungen sind auf einer untersten Reaktorebene angeordnet, die Abkühlvorrichtungen sind unterhalb der Reaktionsvorrichtungen angeordnet, und die Kondensationsvorrichtungen sind oberhalb der Abscheidevorrichtungen angeordnet.

Eine bevorzugte Ausführungsform des erfindungsgemäßen Reaktors ist dadurch gekennzeichnet, dass die Abkühlvorrichtung als Gas-Gas-Wärmeaustauscher ausgestaltet ist, und wobei der Gas-Gas-Wärmeaustauscher so konfiguriert ist, dass die Abkühlung des aus der Reaktionsvorrichtung austretenden Produktgases im Gas-Gas-Wärmeaustauscher gegen einen Strom des Einsatzgasgemischs oder gegen einen Strom des Restgasgemischs erfolgt, insbesondere durch Gravitation.

Der Gas-Gas-Wärmeaustauscher ist insbesondere als indirekter Wärmeaustauscher ausgestaltet.

Gemäß dieser Ausführungsform wird die Wärmeintegration des Reaktors optimiert, indem das am festen Katalysator umzusetzende Einsatzgasgemisch oder Restgasgemisch durch den Strom des aus der jeweiligen Reaktionsvorrichtung austretenden Produktgasgemischs vorgewärmt wird. Da in jeder Reaktionszelle nach der Abkühlung in der Abkühlstufe eine weitere Abkühlung des Restgasgemischs in der Phasentrennvorrichtung erfolgt, insbesondere in der Kondensationsvorrichtung einer Phasentrennvorrichtung erfolgt, kann dieses gekühlte Restgasgemisch erneut als Kühlmedium in einer nachgeschalteten Reaktionszelle zum Abkühlen des aus dieser nachgeschalteten Reaktionszelle austretenden Produktgasgemischs verwendet werden.

Eine bevorzugte Ausführungsform des erfindungsgemäßen Reaktors ist in Folge dadurch gekennzeichnet, dass der Reaktor so konfiguriert ist, dass das Einsatzgasgemisch über den Gas-Gas-Wärmeaustauscher der ersten Reaktionszelle in den Reaktor eintritt, und dabei von dem aus der Reaktionsvorrichtung der ersten Reaktorzelle austretenden Produktgasgemisch aufgeheizt wird.

Vorzugsweise ist der der Gas-Gas-Wärmeaustauscher dabei so konfiguriert, dass der Strom des Produktgasgemischs innerhalb des Gas-Gas-Wärmeaustauschers von oben nach unten strömt und der Strom des Einsatzgasgemischs oder des Restgasgemischs innerhalb des Gas-Gas-Wärmeaustauschers von unten nach oben strömt.

Allgemein werden durch die vertikale Anordnung der Reaktorzellen (Reaktionsvorrichtungen oben, Vorrichtungen zum Kühlen und Kondensieren weiter unten) und die Nutzung des Gegenstromprinzips die folgenden Vorteile erzielt. Zum einen wird eine optimierte Wärmeübertragung zwischen den Prozessströmen durch die Nutzung des Gegenstromprinzips erzielt. Durch das Erwärmen in Aufwärtsrichtung wird ferner die höchste Temperatur am Kopf des Reaktors erzielt, also dort wo die Reaktion des Einsatzgasgemischs erfolgen soll. Gleichzeitig wird in Abwärtsrichtung gekühlt, so dass die Temperaturen im Sumpfbereich des Reaktors am niedrigsten sind, also dort, wo die Kondensation des flüssigen Reaktionsprodukts erfolgen soll. Dies führt zwangsläufig und in vorteilhafter Weise auch zu einem Kondensieren in Abwärtsrichtung, wodurch eine natürliche Kondensat-Flussrichtung ermöglicht wird.

Eine bevorzugte Ausführungsform des erfindungsgemäßen Reaktors ist dadurch gekennzeichnet, dass der Gas-Gas-Wärmeaustauscher als Thermoblech-Wärmeübertrager ausgestaltet ist, und der Thermoblech-Wärmeübertrager so konfiguriert ist, dass das Einsatzgasgemisch oder Restgasgemisch den Thermoblech-Wärmeübertrager innerhalb der Thermobleche durchströmt und der Produktstrom den Thermoblech-Wärmeübertrager zwischen den Thermoblechen durchströmt.

Thermoblech-Wärmeübertrager werden alternativ auch als Kissenplatten-Wärmeübertrager (engl. *pillow plate heat exchanger*) bezeichnet. Entsprechend können die Thermobleche auch als Kissenplatten bezeichnet werden. Die Thermobleche sind insbesondere parallel zueinander angeordnet. Die Thermobleche sind durchströmbare Kissenplatten. Zwischen zwei insbesondere parallel zueinander angeordneten Thermoblechen befindet sich ein durchströmbarer Zwischenraum.

Thermoblech-Wärmeübertrager ermöglichen eine besonders platzsparende Anordnung der Gas-Gas-Wärmeaustauschers innerhalb des Reaktors. Weiterhin ergeben sich durch die Verwendung von Thermoblech-Wärmeübertragern Vorteile hinsichtlich des prozessseitigen Druckverluste. Durch die Verwendung von Thermoblechen lassen sich grundsätzlich geringe Druckverluste bei gleichzeitig hohen Wärmeübertragungskoeffizienten gegenüber konventionellen Platten-Wärmeübertragern oder Rohrbündel-Wärmeübertragern erzielen.

Eine bevorzugte Ausführungsform des erfindungsgemäßen Reaktors ist daher dadurch gekennzeichnet, dass die Reaktionsvorrichtung als Thermoblech-Wärmeübertrager ausgestaltet ist, und der Thermoblech-Wärmeübertrager so konfiguriert ist, dass das Kühlmedium der Reaktionsvorrichtung den Thermoblech-Wärmeübertrager innerhalb der Thermobleche durchströmt und der feste Katalysator zwischen den Thermoblechen des Thermoblech-Wärmeübertragers angeordnet ist, so dass das Einsatzgasgemisch den Thermoblech-Wärmeübertrager zwischen den Thermoblechen durchströmt.

Eine weitere bevorzugte Ausführungsform des erfindungsgemäßen Reaktors ist daher dadurch gekennzeichnet, dass die Kondensationsvorrichtung als Thermoblech-Wärmeübertrager ausgestaltet ist, und der Thermoblech-Wärmeübertrager so konfiguriert ist, dass das Kühlmedium der Kondensationsvorrichtung den Thermoblech-Wärmeübertrager innerhalb der Thermobleche durchströmt, und das zu kondensierende Produktgasgemisch und das Restgasgemisch den Thermoblech-Wärmeübertrager zwischen den Thermoblechen durchströmt.

Gemäß einer bevorzugten Ausführungsform ist die Reaktionsvorrichtung entsprechend der obigen Konfiguration als Thermoblech-Wärmeübertrager ausgestaltet, und/oder die Abkühlvorrichtung, insbesondere der Gas-Gas-Wärmeaustauscher gemäß obiger Konfiguration, ist als Thermoblech-Wärmeübertrager ausgestaltet, und/oder die Kondensationsvorrichtung ist gemäß obiger Konfiguration als Thermoblech-Wärmeübertrager ausgestaltet.

Dabei sind vorzugsweise zumindest zwei hintereinander geschaltete Reaktorzellenelemente innerhalb einer Reaktorzelle als Thermoblech-Wärmeübertrager ausgestaltet, beispielsweise die Reaktionsvorrichtung und die Abkühlvorrichtung. Möglich ist auch, dass alle Reaktorzellenelemente als Thermoblech-Wärmeübertrager ausgestaltet sind, bei denen dies möglich ist, beispielsweise die Reaktionsvorrichtung, die Abkühlvorrichtung und die Kondensationsvorrichtung. Dadurch ergeben sich bei entsprechender Anordnung der Thermoblech-Wärmeübertrager Strömungswege in einer Flucht innerhalb jeder Reaktorzelle, und zwar im besten Fall in Bezug auf die Vorwärmung des Einsatzgasgemischs oder Restgasgemischs, die eigentliche Reaktion, und die Abkühlung sowie die Kondensation des Produktgasgemischs. Dadurch werden Strömungsverluste und Strömungswiderstände weiter reduziert, wodurch auch der Druckverlust weiter minimiert wird. Trotz kompakter Bauweise des Reaktors sind dadurch nur sehr wenige Umlenkungen der gasförmigen und flüssigen Reaktionsmedien erforderlich.

Die Reaktionsvorrichtungen weisen jeweils ein erstes Ende und ein zweites Ende auf, und sind so konfiguriert, dass das Einsatzgasgemisch und/oder das Restgasgemisch am jeweils ersten Ende in die jeweilige Reaktionsvorrichtung eintritt und am jeweils zweiten Ende aus der jeweiligen Reaktionsvorrichtung austritt. Die Kühlvorrichtung der Reaktionsvorrichtungen ist vorzugsweise so konfiguriert, dass ein Eintrittsstutzen der Kühlvorrichtung in einem Bereich des ersten Endes oder in einem Bereich des zweiten Endes der Reaktionsvorrichtungen angeordnet ist. Ist der Eintrittsstutzen der Kühlvorrichtung in einem Bereich des ersten Endes der Reaktionsvorrichtungen angeordnet, tritt das Kühlmedium vorzugsweise erst am zweiten Ende einer jeweiligen Reaktionsvorrichtung in Wärmeaustauschbeziehung mit dem festen Katalysator. Mit anderen Worten, das Kühlmedium strömt gemäß dieser Ausführungsform zunächst vom ersten Ende der Reaktionsvorrichtung zu ihrem zweiten Ende, ohne dass das Reaktionsgemisch dabei gekühlt oder dessen Temperatur durch Wärmeübertragung kontrolliert wird. Die Kühlvorrichtung weist in diesem Fall somit eine Fluidverbindung zwischen dem Eintrittsstutzen und dem zweiten Ende der jeweiligen Reaktionsvorrichtung auf, welche nicht in Wärmeaustauschbeziehung mit dem festen Katalysator steht.

Das Kühlmedium kann am Austrittsstutzen eine höhere Temperatur aufweisen als am Eintrittsstutzen. Insbesondere tritt das Kühlmedium als siedendes Kesselspeisewasser am Eintrittsstutzen in den Reaktor ein und als Dampf am Austrittsstutzen wieder aus.

Die Kühlvorrichtung weist dabei optional ein Verteilersystem für die Zuführung des Kühlmediums und ein Sammlersystem für das Abführen des erhitzten Kühlmediums auf. Der Verteiler hat dabei die Aufgabe, frisches Kühlmedium, insbesondere siedendes Kesselspeisewasser, nach Eintritt über den Eintrittsstutzen auf die jeweiligen Teile der Kühlvorrichtung für die Reaktionsvorrichtungen der unterschiedlichen Reaktorzellen zu verteilen. Dem Sammlersystem kommt die Aufgabe zuteil, erhitztes Kühlmedium, insbesondere Dampf, nach Austritt aus den jeweiligen Teilen der Kühlvorrichtung für die Reaktionsvorrichtungen der unterschiedlichen Reaktorzellen zusammenzuführen. Der zusammengeführte Dampf tritt anschließend über den Austrittsstutzen der Kühlvorrichtung aus dem Reaktor aus.

Eine bevorzugte Ausführungsform des erfindungsgemäßen Reaktors ist dadurch gekennzeichnet, dass die Reaktionsvorrichtung einer Reaktorzelle eine Mehrzahl von Kompartimenten aufweist, wobei ein erstes Kompartiment nicht mit Katalysator befüllt ist und so konfiguriert ist, dass es von unten nach oben vom Einsatzgasgemisch oder Restgasgemisch durchströmbar ist, und wobei zum ersten Kompartiment stromabwärts angeordnete Kompartimente mit Katalysator befüllt sind und so konfiguriert sind, dass sie von oben nach unten vom Einsatzgasgemisch oder Restgasgemisch durchströmbar sind.

Vorzugsweise weist die Reaktionsvorrichtung einer Reaktorzelle eine Mehrzahl, das heißt mindestens zwei räumlich voneinander getrennte Kompartimente auf. Dabei ist das in Gasflussrichtung zuerst durchströmte Kompartiment, also das erste Kompartiment, vorzugsweise nicht mit Katalysator befüllt. Dieses Kompartiment dient in erster Linie dazu, das in der Abkühlvorrichtung, insbesondere im Gas-Gas-Wärmeaustauscher, vorgeheizte Einsatzgasgemisch oder Restgasgemisch an den Kopf des Reaktors, insbesondere an den Kopf der jeweiligen Reaktionsvorrichtung, zu transportieren. Nach einer Umlenkung dieses noch nicht umgesetzten Stroms von Einsatzgasgemisch oder Restgasgemisch durchströmt dieser das Reaktorbett des festen Katalysators vorzugsweise, bei vertikaler Anordnung des Reaktors, von oben nach unten. Dabei wird das dem ersten Kompartiment in Gasflussrichtung nachgeschaltete oder, wenn mehr als zwei Kompartimente vorhanden sind, die dem ersten Kompartiment in Gasflussrichtung nachgeschalteten Kompartimente, in der gleichen Richtung vom Einsatzgasgemisch oder Restgasgemisch durchströmt, wobei das Einsatzgasgemisch oder Restgasgemisch am festen Katalysator zu Produktgasgemisch umgesetzt wird. Gleichzeitig wird das sich durch die exotherme Reaktion aufheizende Gasgemisch im Gegenstrom durch das Kühlmedium der jeweiligen Reaktionsstufe, insbesondere siedendes Kesselspeisewasser, gekühlt. Der sich dabei auf der Kühlseite der Reaktionsstufe bildende Dampf strömt bei vertikaler Anordnung des Reaktors nach oben, was eine natürliche Dampfflussrichtung ermöglicht. Wäre auch das erste Kompartiment mit festem Katalysator gefüllt, so würde die Kühlung des Produktgasgemischs im ersten Kompartiment im Gleichstrom ablaufen, was keine effiziente Kühlung erlaubt. Oder die Flussrichtung des Kühlmediums müsste für das erste Kompartiment umgekehrt werden, was den Aufbau des Reaktors unnötig verkomplizieren würde.

Gemäß einem weiteren Aspekt der Erfindung wird die Verwendung des erfindungsgemäßen Reaktors gemäß einer der vorgenannten Ausführungsformen zur Herstellung von Methanol aus Synthesegas vorgeschlagen, wobei das Synthesegas Kohlenstoffoxide (CO, CO₂) und Wasserstoff (H₂) aufweist.

Gemäß einer Ausführungsform der erfindungsgemäßen Verwendung weist das Synthesegas kein Kohlenmonoxid oder kein Kohlendioxid auf.

Im Hinblick auf die Reduzierung von Kohlendioxidemissionen werden insbesondere kohlenmonoxidfreie Synthesegase in Zukunft immer wichtiger. Synthesegasgemische zur Herstellung von Methanol, welche Kohlendioxid und Wasserstoff als Hauptkomponente erhalten, sind beispielsweise durch Mischen von Kohlendioxid aus Industrieabgasgen, zum Beispiel Müllheizkraftwerken, und klimaneutralem Wasserstoff (sogenanntem "blauem" Wasserstoff) möglich. Klimaneutraler Wasserstoff kann beispielsweise durch Elektrolyse von Wasser durch klimaneutralen Strom, zum Beispiel aus Windkraft, produziert werden.

### Ausführungsbeispiel

Die Erfindung wird im Folgenden durch ein Ausführungsbeispiel näher erläutert. In der folgenden detaillierten Beschreibung wird auf die beigefügten Zeichnungen verwiesen, die einen Teil des Ausführungsbeispiels bilden und in welcher illustrativ eine spezifische Ausführungsform der Erfindung dargestellt ist. In diesem Zusammenhang wird richtungsspezifische Terminologie wie "oben", "unten", "vorne", "hinten" usw. in Bezug auf die Ausrichtung der beschriebenen Figur verwendet. Da Komponenten von Ausführungsformen in einer Vielzahl von Ausrichtungen positioniert werden können, dient die richtungsspezifische Terminologie zur Veranschaulichung und ist in keiner Weise limitierend. Der Fachmann ist sich darüber im Klaren, dass andere Ausführungsformen verwendet werden können und strukturelle oder logische Änderungen vorgenommen werden können, ohne vom Schutzumfang der Erfindung abzuweichen. Die folgende detaillierte Beschreibung ist daher nicht in einem einschränkenden Sinne zu verstehen, und der Schutzumfang der Ausführungsformen wird durch die beigefügten Ansprüche definiert. Die Zeichnungen sind, soweit nicht anders angegeben, nicht zwangsläufig maßstabsgetreu.

In der folgenden Beschreibung und in den Zeichnungen sind gleiche Elemente mit gleichen Bezugszeichen bezeichnet. Pfeile, soweit nicht mit Bezugszeichen versehen, illustrieren die Strömungsrichtung des Einsatzgasgemisches, Produktgasgemischs, Restgasgemischs, flüssigen Reaktionsproduktes oder Kombinationen davon innerhalb des erfindungsgemäßen Reaktors.

Es zeigen
- Figur 1: eine Prinzipskizze einer Ausführungsform des erfindungsgemäßen Reaktors, und
- Figur 2: eine schematische Darstellung für ein Beispiel einer mechanischen Ausgestaltung des erfindungsgemäßen Reaktors.

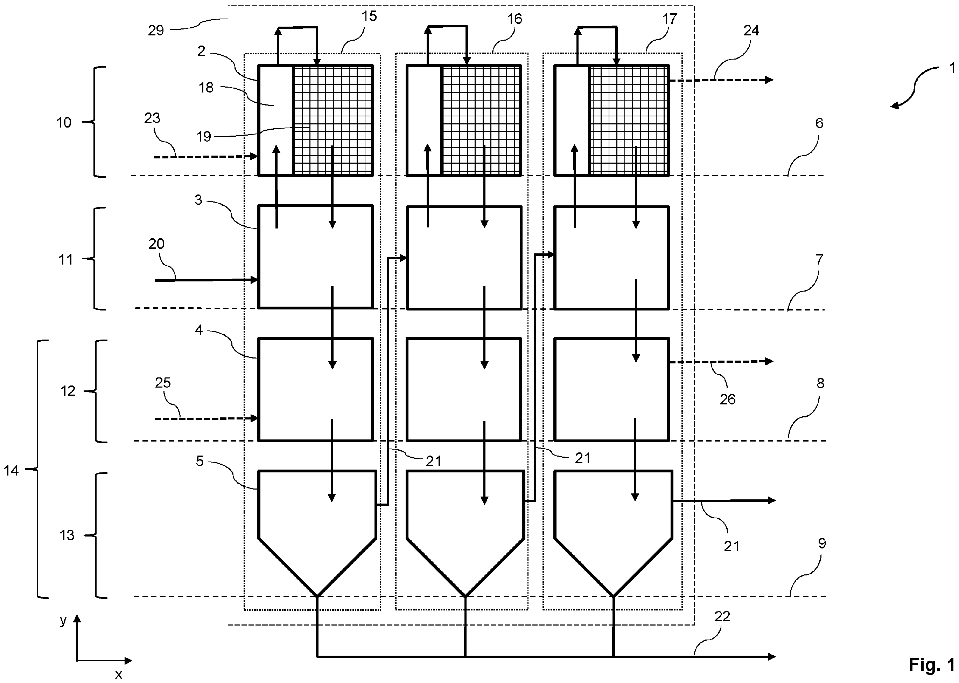

Figur 1 zeigt eine stark vereinfachte schematische Darstellung (Prinzipskizze) einer Ausführungsform des erfindungsgemäßen Reaktors, anhand welcher insbesondere die Anordnung der einzelnen Reaktorelemente und der Strömungsverlauf im Reaktor erläutert werden soll.

Der Reaktor 1 verfügt über einen Reaktormantel 29, welcher alle wesentlichen Reaktorzellenelemente des Reaktors 1 umgibt. Zu- und Ableitungen oder Stutzen für dieselben können sich durch den Reaktormantel hindurch erstrecken.

Der Reaktor 1 gemäß Figur 1 eignet sich zur Herstellung von Methanol aus Synthesegas als Einsatzgas. Synthesegas ist im Beispiel ein Gemisch aus Wasserstoff, Kohlenmonoxid und Kohlendioxid. Bei der Umsetzung des Einsatzgases am festen Katalysator des Reaktors 1 entsteht ein Produktgasgemisch und ein Restgasgemisch. Das Produktgasgemisch enthält Methanol und Wasser, und das Restgasgemisch enthält nicht umgesetztes Synthesegas. Das Produktgasgemisch kann darüber hinaus gehend kondensierbare Nebenprodukte, wie zum Beispiel höhere Alkohole, Ketone, Ether und Ester enthalten. Das Restgasgemisch kann nicht kondensierbare Nebenprodukte und nicht umsetzbare Bestandteile aus dem Syntheserohgas, wie zum Beispiel Methan und Stickstoff, enthalten.

Der Reaktor 1 umfasst insgesamt drei Reaktorzellen, welche gemäß Figur 1 als erste Reaktorzelle 15, zweite Reaktorzelle 16 und dritte Reaktorzelle 17 bezeichnet sind. Jede der Reaktorzellen weist vier verschiedene Reaktorzellenelemente auf. Bei den Reaktorzellenelementen je Reaktorzelle 15, 16 oder 17 handelt es sich, in der Zeichnung von oben nach unten angeordnet, um eine Reaktionsvorrichtung 2, eine Abkühlvorrichtung 3, eine Kondensationsvorrichtung 4 und eine Abscheidevorrichtung 5. Der Reaktor 1 weist vier parallel zueinander angeordnete Reaktorebenen auf. Von oben nach unten angeordnet wie in der Zeichnung dargestellt handelt es sich dabei um die Reaktorebenen 6, 7, 8 und 9. Reaktorzellenelemente der gleichen Art sind dabei jeweils auf einer gemeinsamen oder derselben Reaktorebene angeordnet. So befinden sich auf der ersten Reaktorebene angeordnet insgesamt drei Reaktionsvorrichtungen 2, welche zusammengefasst als Reaktionsvorrichtungen 10 auf der ersten Reaktorebene 6 dargestellt sind. Ferner befinden sich auf der zweiten Reaktorebene angeordnet insgesamt drei Abkühlvorrichtungen 3, welche zusammengefasst als Abkühlvorrichtungen 11 auf der zweiten Reaktorebene 7 dargestellt sind. Ferner befinden sich auf der dritten Reaktorebene angeordnet insgesamt drei Kondensationsvorrichtungen 4, welche zusammengefasst als Kondensationsvorrichtungen 12 auf der dritten Reaktorebene 8 dargestellt sind. Ferner befinden sich auf der vierten Reaktorebene angeordnet insgesamt drei Abscheidevorrichtungen 5, welche zusammengefasst als Abscheidevorrichtungen 13 auf der vierten Reaktorebene 9 dargestellt sind. Die Kondensationsvorrichtungen 12 und Abscheidevorrichtungen 13 können auch als integrierte Phasentrennvorrichtungen ausgestaltet sein, welche dann als Phasentrennvorrichtungen 14 auf einer dann dritten Reaktorebene angeordnet sind, wobei diese dritte Reaktorebene in der Darstellung in Figur 1 der Reaktorebene 9 entspräche. Die Reaktorebenen 6, 7, 8 und 9 sind gemäß der dargestellten Ausführungsform nicht als dingliche Ebenen, sondern lediglich als virtuelle Ebenen zu verstehen. Mit anderen Worten sind die Reaktorzellenelemente derselben Art in einer Linie auf dieser Ebene angeordnet. So ergibt sich beispielsweise bei vertikaler Anordnung des Reaktors 1, dass die Reaktorzellenelemente derselben Art durch Anordnung auf derselben Reaktorebene auf jeweils der gleichen Höhe oder im Wesentlichen auf der gleichen Höhe angeordnet sind. Verläuft die Hauptachse des Reaktors 1 wie in Figur 1 dargestellt im Sinne eines kartesischen Koordinatensystems entlang der y-Achse, so wird die Ausdehnung einer Reaktorebene 6, 7, 8 oder 9 über eine Fläche definiert, welche sich in x-z-Richtung erstreckt. Die z-Achse verläuft dabei senkrecht zur dargestellten Zeichenebene. Die Positionierung der sich in x-z-Richtung erstreckenden Reaktorebenen 6, 7, 8 oder 9, beispielsweise deren Höhe, wird dabei durch deren Position an der y-Achse, also an der Position der Hauptachse des Reaktors, definiert.

Durch die dargestellte und erfindungsgemäße Anordnung der Reaktorzellenelemente wird erreicht, dass Reaktorzellenelemente unterschiedlicher Art, welche im Betrieb gleichzeitig große Temperaturunterschiede zueinander aufweisen, nicht nebeneinander angeordnet sind. Bei einer rein linearen Anordnung aller drei Reaktorzellen 15, 16 und 17, mit vertikaler oder horizontaler Ausrichtung der Hauptachse, wäre beispielsweise die Reaktionsvorrichtung 2 der zweiten Reaktorzelle 16 neben der Abscheidevorrichtung 5 der ersten Reaktionszelle 15 angeordnet. Dies würde einen hohen Aufwand für die thermische Isolation zumindest eines der Reaktorzellenelemente erfordern, da die Abwärme der Reaktionsvorrichtung sonst die Leistungsfähigkeit und damit Produktausbeute der Abscheidevorrichtung stark beinträchtigen würde. Weiterhin wäre dadurch mit einem hohen Maß an Materialspannungen zwischen der heißen Reaktionsvorrichtung und der kalten Abscheidevorrichtung zu rechnen, welche beide miteinander in Fluidverbindung stehen und dadurch zwangsläufig über Leitungen miteinander verbunden sind.

Die Abkühlvorrichtungen 11 sind im Beispiel gemäß Figur 1 als Gas-Gas-Wärmeaustauscher ausgestaltet. Dabei verfügt die Abkühlvorrichtung 3 der ersten Reaktorzelle 15 über einen Einsatzgaseinlass 20 für den Eintritt des frischen Synthesegases. In der ersten Abkühlvorrichtung 3 der ersten Reaktorzelle 15 wird das Einsatzgas gegen das aus der Reaktionsvorrichtung 2 austretende Produktgasgemisch und Restgasgemisch durch indirekten Wärmeaustausch aufgeheizt, wobei das Produktgasgemisch und Restgasgemisch in der Abkühlvorrichtung abgekühlt wird. Nach dem Aufheizen des Einsatzgasgemischs in der Abkühlvorrichtung 3 der ersten Reaktorzelle 15 tritt dieses in die Reaktionsvorrichtung 2 ein. Die Reaktionsvorrichtung 2 enthält, wie alle Reaktionsvorrichtungen 10 auf der ersten Reaktorebene 6, einen festen Katalysator auf beispielsweise Kupfer-Basis zur Umsetzung des Einsatzgasgemischs zu Methanol und Wasser. Jede der Reaktionsvorrichtungen 2 weist darüber hinaus zumindest zwei unterschiedliche Kompartimente 18 und 19 auf, wobei das in Strömungsrichtung zuerst angeordnete, erste Kompartiment 18 keinen Katalysator enthält, im ersten Kompartiment 18 somit keine Reaktion stattfindet. Das in Strömungsrichtung zumindest zweite angeordnete Kompartiment, im gezeigten Fall die weiteren Kompartimente 19, sind mit dem festen Katalysator befüllt. In den weiteren Kompartimenten 19 findet somit die Reaktion des Einsatzgasgemischs zu Methanol und Wasser statt. Der feste Katalysator in den weiteren Kompartimenten 19 steht in Wärmeaustauschbeziehung mit einer Kühlvorrichtung der Reaktionsvorrichtung 2, welche mit siedendem Kesselspeisewasser als Kühlmedium betrieben wird. Die Kühlvorrichtung dient dabei vornehmlich der Temperaturkontrolle der exothermen Reaktion, insbesondere der Einhaltung einer maximal zulässigen Temperatur am Katalysatorbett des festen Katalysators. Die Reaktionsvorrichtung 2 verfügt daher über einen Kesselspeisewassereinlass 23. Das eintretende Kesselspeisewasser kühlt das sich durch die exotherme Reaktion aufheizende Produktgasgemisch und Restgasgemisch im indirekten Wärmeaustausch gemäß dem Prinzip der Siedekühlung. Das siedende Kesselspeisewasser strömt in der Reaktionsvorrichtung 2 dabei von unten nach oben. Einsatzgasgemisch strömt im ersten Kompartiment 18 von unten nach oben, also in gleicher Strömungsrichtung wie das Kühlmedium, wobei jedoch keine Kühlung des Einsatzgases im ersten Kompartiment 18 stattfindet. Das Einsatzgas wird anschließend umgelenkt und strömt dann in den mit Katalysator befüllten weiteren Kompartimenten 19 in der Reaktionsvorrichtung 2 von oben nach unten, wobei es mit im Gegenstrom dazu geführtem siedendem Kesselspeisewasser indirekt gekühlt wird. Die Strömungsführung ist innerhalb des Reaktormantels 29 des Reaktors 1 so ausgestaltet, dass in jeder der Reaktionsvorrichtungen 3 Kesselspeisewasser von unten nach oben strömt und am oberen Ende einer jeden Reaktionsvorrichtung 3 diese als Sattdampf verlässt. Der erfindungsgemäße Reaktor 1 verfügt über ein entsprechendes Verteiler- und Sammlersystem, welches diese Strömungsführung ermöglicht. In jeder der Reaktionsvorrichtungen 3 wird so das sich am Katalysator aufheizende Produktgasgemisch und Restgasgemisch im Gegenstrom indirekt mit siedendem Kesselspeisewasser gekühlt. Das durch den Sammler zusammengeführte und aufgeheizte Kühlmedium, der Sattdampf, verlässt den Reaktor am Sattdampfauslass 24 und kann als Exportdampf oder prozessintern, beispielsweise in einem vorgeschalteten Dampfreformer, genutzt werden.

Das Produktgasgemisch und das nicht umgesetzte Einsatzgasgemisch, also Restgasgemisch, treten anschließend in die Abkühlvorrichtung 3 der ersten Reaktorzelle 15 ein und werden dort, wie oben beschrieben, im Gegenstrom gegen Einsatzgasgemisch abgekühlt. Dabei erfolgt weitestgehend noch keine Kondensation des Produktgasgemischs zum flüssigen Reaktionsprodukt, eine Teilkondensation in der Abkühlvorrichtung 3 ist jedoch möglich. Das vollständig gasförmige oder teilkondensierte Gemisch tritt anschließend in die Kondensationsvorrichtung 4 ein, in welcher das Produktgasgemisch vollständig oder im Wesentlichen vollständig kondensiert wird, so dass flüssiges Reaktionsprodukt, ein Gemisch aus Methanol und Wasser und kondensierten unerwünschten Nebenprodukten, sowie gasförmiges Restgasgemisch in der Kondensationsvorrichtung 4 anfallen. Die Kühlung in der Kondensationsvorrichtung 4 der ersten Reaktorzelle 15 sowie den weiteren Reaktorzellen 16 und 17 erfolgt mit Kühlwasser durch indirekten Wärmeaustausch mit dem kondensierenden Gemisch im Gegenstrom. Der Reaktor 1 verfügt daher über einen Kühlwassereinlass 25 und einen Kühlwasserauslass 26 an den Kondensationsvorrichtungen 12 der Reaktorebene 8. Dabei verfügt der Reaktor 1 auch bezüglich des Kühlsystems in den Kondensationsvorrichtungen 12 über ein entsprechendes Verteiler- und Sammlersystem für das für die Kondensation verwendete Kühlwasser, so dass in jeder der Kondensationsvorrichtungen 4 frisches Kühlwasser über einen Verteiler (nicht gezeigt) zur Verfügung steht. Verbrauchtes Kühlwasser wird im Sammler zusammengeführt und über den Kühlwasserauslass 26 aus dem Reaktor 1 ausgeleitet.

Das Gemisch aus kondensiertem flüssigem Reaktionsprodukt (Methanol, Wasser, kondensierte Nebenprodukte) und gasförmigem Restgasgemisch (Wasserstoff, Kohlendioxid, Kohlenmonoxid, Methan, Stickstoff, nicht kondensierte Nebenprodukte) wird in der Abscheidevorrichtung 5 der ersten Reaktorzelle 15 in den entsprechenden flüssigen Produktteil und in einen gasförmigen Teil getrennt. Abscheidevorrichtung 5 ist beispielsweise als Gas-Flüssigkeits-Abscheider ausgestaltet. Das flüssige Reaktionsprodukt, im Wesentlichen Methanol und Wasser, wird aus der Abscheidevorrichtung 5 ausgeleitet. Die flüssigen Reaktionsprodukte der Abscheidevorrichtungen 13 auf Reaktorebene 9 werden zusammengeführt und als flüssiges Reaktionsprodukt 22 aus dem Reaktor 1 ausgeleitet. Das Zusammenführen der flüssigen Reaktionsprodukte jeder Reaktorzelle kann dabei innerhalb oder außerhalb des Reaktormantels 29 erfolgen, im Beispiel gemäß Figur 1 außerhalb. Das flüssige Reaktionsprodukt wird anschließend der erforderlichen Aufarbeitung zur Gewinnung von reinem Methanol zugeführt.

Die Abscheidevorrichtung 5 der ersten Reaktorzelle 15 verfügt über einen Restgasgemischauslass 21. Das in der Abkühlvorrichtung 3 und Kondensationsvorrichtung 4 abgekühlte Restgasgemisch wird über den Restgasgemischauslass 21 aus der Abscheidevorrichtung 5 abgezogen und in die Abkühlvorrichtung 3 der zweiten Reaktorzelle 16 eingeschleust, wo es entsprechend den Abläufen in der Abkühlvorrichtung 3 der ersten Reaktorzelle 15 im Gegenstrom gegen Produktgasgemisch und Restgasgemisch aus der Reaktionsvorrichtung 2 der zweiten Reaktorzelle 16 durch indirekten Wärmeaustausch aufgeheizt wird. Das aufgeheizte Restgasgemisch tritt anschließend in das erste, leere Kompartiment der Reaktionsvorrichtung 2 der zweiten Reaktorzelle 16 ein, wird umgelenkt und in den weiteren Kompartimenten 19 der Reaktionsvorrichtung 2 der zweiten Reaktorzelle 16 am festen Katalysator zu Produktgasgemisch und Restgasgemisch umgesetzt. Die weiteren Vorgänge innerhalb der zweiten Reaktorzelle 16 und der dritten Reaktorzelle 17 entsprechen weitestgehend den oben beschriebenen Abläufe bezüglich der ersten Reaktorzelle 15.

In der dritten Reaktorzelle 17 nicht umgesetztes Synthesegas, also Restgasgemisch, verlässt den Reaktor am (dritten) Restgasgemischauslass 21 der Abscheidevorrichtung 5 dieser dritten Reaktorzelle 17. An diesem Restgasgemischauslass 21 abgezogenes Restgasgemisch kann gemäß einer Ausführungsform mit Einsatzgasgemisch zusammengeführt und so weiter verwertet werden. Um die Akkumulation von inerten Bestandteilen im System zu verhindern, kann dem aus der dritten Reaktorzelle abgezogenen Restgasgemisch ein Anteil als Spülgas (Purgegas) entnommen werden. Das Spülgas kann in einem Beispiel als Brenngas in einem vorgeschalteten Dampfreformer verwendet werden, oder es wird einer Druckwechseladsorptionvorrichtung zur Gewinnung von Wasserstoff zugeführt.

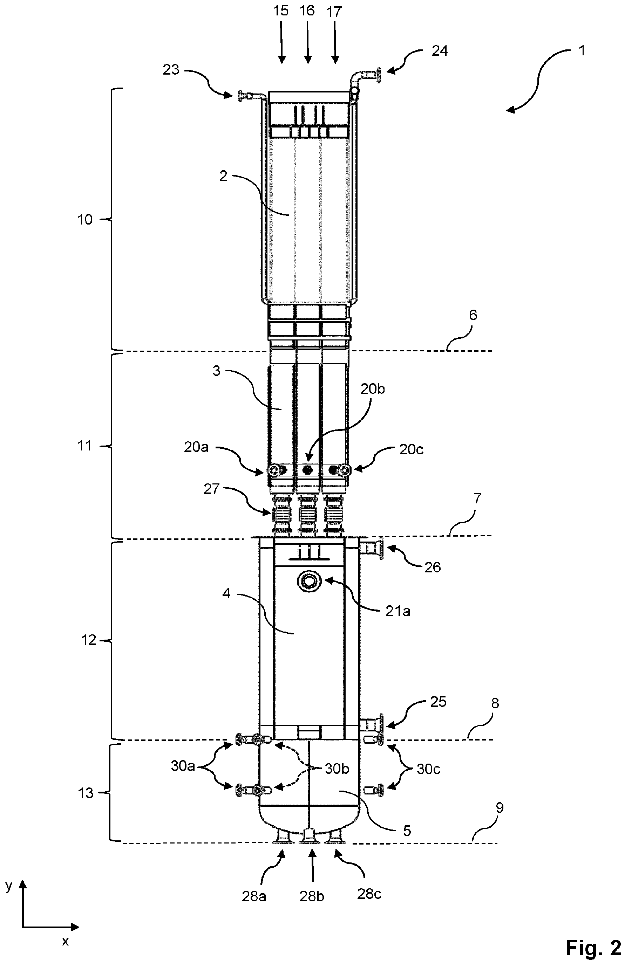

Figur 2 zeigt ein Beispiel für eine mechanische Ausgestaltung des erfindungsgemäßen Reaktors 1 gemäß Figur 1. Der Reaktormantel 29 ist hier zum Zwecke der Vereinfachung und aus Gründen der Übersichtlichkeit nicht dargestellt. Entsprechend der Darstellung in Figur 1 verfügt der Reaktor 1 gemäß Figur 2 über insgesamt drei Reaktorzellen und vier Reaktorebenen. Der Reaktor gemäß Figur 2 verfügt über eine entlang der y-Achse angeordnete Hauptachse, welche hier vertikal verläuft. Von oben nach unten auf den parallel zueinander angeordneten Reaktorebenen 6, 7, 8 und 9 sind dementsprechend jeweils drei Reaktionsvorrichtungen, drei Abkühlvorrichtungen, drei Kondensationsvorrichtungen und drei Abscheidevorrichtungen angeordnet. Entsprechend befinden sich auf der ersten Reaktorebene 6 die Reaktionsvorrichtungen 10, auf der zweiten Reaktorebene 7 die Abkühlvorrichtungen 11, auf der dritten Reaktorebene die Kondensationsvorrichtungen 12 und auf der vierten Reaktorebene 9 die Abscheidevorrichtungen 13. Die vertikale Anordnung der Hauptachse des Reaktors 1 ermöglicht einen natürlichen Fluss des Kondensats von oben nach unten, also von den oberen heißeren Teilen des Reaktors zu den unteren kühleren Teilen des Reaktors. Entsprechendes gilt für den Fluss der sich aufheizenden Kühlmedien, insbesondere den Fluss des siedenden Kesselspeisewassers. Dieses tritt über den Kesselspeisewassereinlass 23 in den Reaktor 1 ein und als Sattdampf über den Sattdampfauslass 24 wieder aus dem Reaktor aus. Dabei erfolgt ein Aufheizen des Kesselspeisewassers innerhalb der Reaktionsvorrichtungen 10 jeweils von unten nach oben. Gleiches gilt für das Kühlwasser in den Kondensationsvorrichtungen 12, welches am Kühlwassereinlass 25 in den Reaktor 1 eintritt, in den Kondensationsvorrichtungen 12 jeweils von unten nach oben strömt und den Reaktor 1 am Kühlwasserauslass 26 in aufgeheizter Form verlässt.

Das Einsatzgas tritt über den Einsatzgasgemischeinlass 20a an der Abkühlvorrichtung 3 der ersten Reaktorzelle 15 in den Reaktor 1 ein und wird in der Reaktionsvorrichtung 2 der ersten Reaktorzelle 15, entsprechend der Beschreibung zur Figur 1, umgesetzt. Das Produktgasgemisch wird anschließend in der Abkühlvorrichtung 2 der ersten Reaktorzelle 15 abgekühlt, in der Kondensationsvorrichtung 4 der ersten Reaktorzelle 15 kondensiert und in der Abscheidevorrichtung 5 der ersten Reaktorzelle 15 als flüssiges Reaktionsprodukt abgeschieden. In der Abscheidevorrichtung 5 der ersten Reaktorzelle 15 abgeschiedenes Restgas tritt über den Restgasgemischauslass 21a aus. Es wird anschließend entweder über eine Rohrleitung innerhalb des Reaktormantels 29 oder außerhalb des Reaktormantels 29 zum Restgasgemischeinlass 20b geführt.

Die externe Führung der Rohrleitungen, also außerhalb des Reaktormantels 29, hat den Vorteil dass für analytische Zwecke leicht Proben aus der Leitung entnommen werden können. Weiterhin wird dadurch die Möglichkeit eröffnet, die drei Reaktorzellen 15, 16 und 17 parallel zu betreiben. Gemäß einer solchen Betriebsweise wird der erfindungsgemäße Reaktor gemäß dem Prinzip einer einstufigen Synthese verwendet. Es könnten dann jedoch drei Reaktoren gleichzeitig betrieben werden, wobei diese Reaktoren hier jeweils durch die Reaktorzellen 15, 16 und 17 repräsentiert werden.

Die interne Führung der Rohrleitungen, also innerhalb des Reaktormantels 29, hat den Vorteil dass insgesamt weniger lange Rohrleitungen erforderlich sind, zwangsläufig auftretende Wärmeverluste geringer sind und weniger potentielle Möglichkeiten für Leckagen vorhanden sind.

Aus dem Restgasgemischauslass 21a austretendes Restgasgemisch tritt anschließend über den Restgasgemischeinlass 20b in die Abkühlvorrichtung 3 der zweiten Reaktorzelle 16 ein, um dort wiederum zunächst aus der Reaktionsvorrichtung 2 der Reaktorzelle 16 austretendes Produktgasgemisch zu kühlen. Dabei wird das Restgasgemisch vorgeheizt und tritt anschließend vorgeheizt am Kopf der Reaktorzelle 16 in die Reaktionsvorrichtung 2 der zweiten Reaktorzelle 16 ein. Nach Umsetzung zum Produktgasgemisch in Reaktionsvorrichtung 2, Kühlung in Kühlvorrichtung 3, Kondensation des flüssigen Reaktionsprodukts in Kondensationsvorrichtung 4 und schließlich Abscheidung in Abscheidevorrichtung 5 der zweiten Reaktorzelle 16 wird verbleibendes Restgasgemisch aus einem weiteren auf Höhe der Kondensationsvorrichtungen 12 angeordneten Restgasgemischauslass abgezogen. Dieser Restgasgemischauslass ist auf der Rückseite des dargestellten Reaktors 1 angeordnet und daher nicht gezeigt (gemäß der verwendeten Bezugszeichensystematik hätte dieser Restgasgemischauslass das Bezugszeichen 21b). Das über diesen Restgasgemischauslass abgezogene Restgas wird entsprechend dem oben gesagten über den Restgasgemischeinlass 20c in die dritte Reaktionszelle 17 eingeschleust. In der Abscheidestufe 5 der dritten und letzten Reaktorzelle 17 abgeschiedenes Restgas wird schließlich über einen letzten Restgasgemischauslass (ebenfalls nicht gezeigt, entspräche Restgasgemischauslass 21c) abgezogen. Dieses Restgas kann optional wieder zum Einsatzgasgemischeinlass 20a geführt werde, wird in diesem Fall also dem Einsatzgasgemisch zugemischt.

Alternativ oder zusätzlich wird das Restgas einer Vorrichtung zur Wasserstoffrückgewinnung zugeführt.

Gemäß einer weiteren Option kann den jeweiligen Restgasgemischen vor Eintritt in die Restgasgemischeinlässe 20b und 20c auch Einsatzgasgemisch zugemischt werden.

Der Reaktor 1 verfügt je Reaktorzelle über einen Auslass für flüssiges Reaktionsprodukt. Entsprechend gezeigt ist der Auslass 28a für die erste Reaktorzelle 15, der Auslass 28b für die zweite Reaktorzelle 16 und der Auslass 28b für die dritte und letzte Reaktorzelle 17. Die Auslässe für das flüssige Reaktionsprodukt 28a, 28b und 28c sind im Bodenbereich des Reaktors 1 beziehungsweise im Bodenbereich der Abscheidevorrichtungen 13 angeordnet. Jede der Abscheidevorrichtungen 13 verfügt über zwei Anschlussstutzen zur Regelung des Füllstands an flüssigem Reaktionsprodukt in der jeweiligen Abscheidevorrichtung 5. Gezeigt sind die Stutzen 30a der Abscheidevorrichtung 5 der ersten Reaktorzelle 15, die Stutzen 30b der Abscheidevorrichtung 5 der zweiten Reaktorzelle 16, und die Stutzen 30c der Abscheidevorrichtung 5 der dritten Reaktorzelle 17.

Zum Ausgleich von thermischen Spannungen insbesondere in Längsrichtung verfügt jede der Abkühlvorrichtungen 11 über einen Dehnungsbalg 27, welcher jeweils stromaufwärts zur folgenden Kondensationsvorrichtung angeordnet ist.

Zumindest die Reaktionsvorrichtungen 10 und die Abkühlvorrichtungen 11 des Reaktors 1 sind als Thermoblech-Wärmeübertrager (Kissenplatten-Wärmeübertrager) ausgestaltet. Dabei durchströmt das Einsatzgasgemisch oder Restgasgemisch die Reaktionsvorrichtung zwischen den einzelnen Thermoblechen, also einen Bereich in dem sich auch das Katalysatorbett des festen Katalysators befindet. Innerhalb der Thermobleche oder Kissenplatten werden die Reaktionsvorrichtungen von dem siedenden Kesselspeisewasser durchströmt. Entsprechend strömt das sich abkühlende Produktgasgemisch und Restgasgemisch die Abkühlvorrichtungen 11 zwischen den Thermoblechen, während das sich aufheizende Einsatzgasgemisch oder Restgasgemisch innerhalb der Thermobleche oder Kissenplatten strömt. Dies ermöglicht eine fluchtende Anordnung der einzelnen Reaktionsvorrichtungen und Abkühlvorrichtungen bezüglich der Räume, welche von Produktgasgemisch und Restgasgemisch oder Kühlmedium (Kesselspeisewasser, Einsatzgasgemisch oder Restgasgemisch) durchströmt werden. Dadurch kann der Reaktor 1 in vorteilhafter Weise besonders kompakt gebaut werden.

### Bezugszeichenliste

- 1: Reaktor
- 2: Reaktionsvorrichtung
- 3: Abkühlvorrichtung
- 4: Kondensationsvorrichtung
- 5: Abscheidevorrichtung
- 6: erste Reaktorebene
- 7: zweite Reaktorebene
- 8: dritte Reaktorebene
- 9: vierte Reaktorebene
- 10: Reaktionsvorrichtungen auf erster Reaktorebene
- 11: Abkühlvorrichtungen auf zweiter Reaktorebene
- 12: Kondensationsvorrichtungen auf dritter Reaktorebene
- 13: Abscheidevorrichtungen auf vierter Reaktorebene
- 14: Phasentrennvorrichtungen
- 15: erste Reaktorzelle
- 16: zweite Reaktorzelle
- 17: dritte Reaktorzelle
- 18: erstes, leeres Kompartiment der Reaktionsvorrichtung
- 19: weitere, mit Katalysator befüllte Kompartimente der Reaktionsvorrichtung
- 20a,b,c: Einsatzgasgemischeinlass oder Restgasgemischeinlass
- 21a: Restgasgemischauslass
- 22: flüssiges Reaktionsprodukt
- 23: Kesselspeisewassereinlass
- 24: Sattdampfauslass
- 25: Kühlwassereinlass
- 26: Kühlwasserauslass
- 27: Dehnungsbalg
- 28a,b,c: Auslass flüssiges Reaktionsprodukt
- 29: Reaktormantel
- 30a,b,c: Stutzen für Füllstandsregelung

## Patentansprüche

1. Reaktor (1) zum Durchführen exothermer Gleichgewichtsreaktionen, in dem ein Einsatzgasgemisch und/oder ein Restgasgemisch an einem festen Katalysator zumindest teilweise umsetzbar ist/sind, wobei ein Produktgasgemisch und ein Restgasgemisch erhältlich sind, wobei das Produktgasgemisch ein bei Reaktionsdruck und unterhalb der Reaktionstemperatur kondensierbares, flüssiges Reaktionsprodukt (22) enthält, aufweisend
einen Reaktormantel (29) und eine innerhalb des Reaktormantels angeordnete Mehrzahl hintereinander geschalteter und in Fluidverbindung miteinander stehender Reaktorzellen (15, 16, 17), wobei
jede Reaktorzelle die folgenden, hintereinander geschalteten und in Fluidverbindung miteinander stehenden Reaktorzellenelemente umfasst:
- Eine Reaktionsvorrichtung (2), konfiguriert zum Umsetzen des Einsatzgasgemischs und/oder des Restgasgemischs zum Produktgasgemisch, aufweisend den festen Katalysator und eine mit dem festen Katalysator in Wärmeaustauschbeziehung stehende und von einem Kühlmedium durchströmbare Kühlvorrichtung;
- Eine Abkühlvorrichtung (3), konfiguriert zum Abkühlen des aus der Reaktionsvorrichtung austretenden Produktgasgemischs;
- Eine Phasentrennvorrichtung (14), konfiguriert zum Kondensieren und Abscheiden des flüssigen Reaktionsprodukts aus dem abgekühlten Produktgasgemisch, wobei die kondensierte, flüssige Phase das Reaktionsprodukt und die gasförmige Phase das Restgasgemisch enthält;
und wobei jede Reaktorzelle
- Mittel zum Ausleiten des Reaktionsprodukts aus der Reaktorzelle umfasst, und
- Mittel zum Ausleiten des Restgasgemischs aus der Reaktorzelle umfasst, und
- Mittel zum Zuführen des Restgasgemischs zu einer, soweit vorhanden, stromabwärts angeordneten Reaktorzelle umfasst;
und wobei der Reaktor
- eine Mehrzahl innerhalb des Reaktormantels und parallel zueinander angeordnete Reaktorebenen (6, 7, 8, 9) aufweist, wobei Reaktorzellenelemente derselben Art auf derselben Reaktorebene angeordnet sind.

2. Reaktor nach Anspruch 1, **dadurch gekennzeichnet, dass** auf jeder der Reaktorebenen ausschließlich eine Art eines Reaktorzellenelements angeordnet ist.

3. Reaktor nach Anspruch 1 oder 2, **dadurch gekennzeichnet, dass** die Zahl der vorhandenen Reaktorebenen mindestens der Zahl der vorhandenen Arten von Reaktorzellenelementen entspricht.

4. Reaktor nach einem der vorherigen Ansprüche, **dadurch gekennzeichnet, dass** die Reaktorzellen vertikal gegenüber der durch die Schwerkraft vermittelten Senkrechten angeordnet sind, so dass Ströme innerhalb der und zwischen den Reaktorzellenelementen von unten nach oben und von oben nach unten führbar sind.

5. Reaktor nach einem der vorhergehenden Ansprüche, **dadurch gekennzeichnet, dass** die Reaktorzellenelemente innerhalb des Reaktormantels gemäß einer zweidimensionalen Matrix mit einer Anzahl y Spalten und einer Anzahl x Zeilen angeordnet sind, wobei die Anzahl y Spalten der Anzahl der Reaktorzellen entspricht, und die Anzahl x Zeilen der Anzahl der unterschiedlichen Arten von Reaktorzellenelementen entspricht, wobei eine Spalte der Matrix eine Reaktorzelle mit jeweils einer der unterschiedlichen Arten an Reaktorzellenelementen umfasst und eine Zeile der Matrix eine Reaktorebene aufweisend eine Reihe aus Reaktorzellenelementen derselben Art umfasst.

6. Reaktor nach einem der vorhergehenden Ansprüche, **dadurch gekennzeichnet, dass** die Reaktorebenen parallel zueinander und hintereinander von oben nach unten angeordnet sind.

7. Reaktor nach einem der vorhergehenden Ansprüche, **dadurch gekennzeichnet, dass** der Reaktor ein erstes Ende und ein zweites Ende aufweist, wobei auf den Reaktorebenen vom ersten Ende aus in Richtung des zweiten Endes Reaktorzellenelemente räumlich gemäß folgender Reihenfolge angeordnet sind: Reaktionsvorrichtung, Abkühlvorrichtung, Phasentrennvorrichtung.

8. Reaktor nach einem der vorhergehenden Ansprüche, **dadurch gekennzeichnet, dass** jede Phasentrennvorrichtung eine Kondensationsvorrichtung (12) und eine Abscheidevorrichtung (13) umfasst, wobei die Kondensationsvorrichtungen und die Abscheidevorrichtungen jeweils auf unterschiedlichen Reaktorebenen angeordnet sind.

9. Reaktor nach Anspruch 8, **dadurch gekennzeichnet, dass** der Reaktor ein erstes Ende und ein zweites Ende aufweist, wobei auf den Reaktorebenen vom ersten Ende aus in Richtung des zweiten Endes Kondensationsvorrichtungen und Abscheidevorrichtungen räumlich gemäß folgender Reihenfolge angeordnet sind: Kondensationsvorrichtungen, Abscheidevorrichtungen.

10. Reaktor nach einem der Ansprüche 7 bis 9, **dadurch gekennzeichnet, dass** der gesamte Reaktor vertikal gegenüber der durch die Schwerkraft vermittelten Senkrechten angeordnet ist und sich das erste Ende oben befindet und das zweite Ende unten befindet.

11. Reaktor nach einem der vorhergehenden Ansprüche, **dadurch gekennzeichnet, dass** die Reaktionsvorrichtungen auf einer obersten Reaktorebene angeordnet sind, die Phasentrennvorrichtungen auf einer untersten Reaktorebene angeordnet sind, und die Abkühlvorrichtungen auf einer Reaktorebene zwischen der obersten und untersten Reaktorebene angeordnet sind.

12. Reaktor nach einem der vorhergehenden Ansprüche, **dadurch gekennzeichnet, dass** die Abkühlvorrichtung als Gas-Gas-Wärmeaustauscher ausgestaltet ist, und wobei der Gas-Gas-Wärmeaustauscher so konfiguriert ist, dass die Abkühlung des aus der Reaktionsvorrichtung austretenden Produktgases im Gas-Gas-Wärmeaustauscher gegen einen Strom des Einsatzgasgemischs oder gegen einen Strom des Restgasgemischs erfolgt.

13. Reaktor nach Anspruch 12, **dadurch gekennzeichnet, dass** der Gas-Gas-Wärmeaustauscher so konfiguriert ist, dass der Strom des Produktgasgemischs innerhalb des Gas-Gas-Wärmeaustauschers von oben nach unten strömt und der Strom des Einsatzgasgemischs oder des Restgasgemischs innerhalb des Gas-Gas-Wärmeaustauschers von unten nach oben strömt.

14. Reaktor nach einem der Ansprüche 12 oder 13, **dadurch gekennzeichnet, dass** der Reaktor so konfiguriert ist, dass das Einsatzgasgemisch über den Gas-Gas-Wärmeaustauscher der ersten Reaktionszelle in den Reaktor eintritt, und dabei von dem aus der Reaktionsvorrichtung der ersten Reaktorzelle austretenden Produktgasgemisch aufgeheizt wird.

15. Reaktor nach einem der Ansprüche 12 bis 14, **dadurch gekennzeichnet, dass** der Gas-Gas-Wärmeaustauscher als Thermoblech-Wärmeübertrager ausgestaltet ist, und der Thermoblech-Wärmeübertrager so konfiguriert ist, dass das Einsatzgasgemisch oder Restgasgemisch den Thermoblech-Wärmeübertrager innerhalb der Thermobleche durchströmt und der Produktstrom den Thermoblech-Wärmeübertrager zwischen den Thermoblechen durchströmt.

16. Reaktor nach einem der vorherigen Ansprüche, **dadurch gekennzeichnet, dass** die Reaktionsvorrichtung als Thermoblech-Wärmeübertrager ausgestaltet ist, und der Thermoblech-Wärmeübertrager so konfiguriert ist, dass das Kühlmedium der Reaktionsvorrichtung den Thermoblech-Wärmeübertrager innerhalb der Thermobleche durchströmt und der feste Katalysator zwischen den Thermoblechen des Thermoblech-Wärmeübertragers angeordnet ist, so dass das Einsatzgasgemisch den Thermoblech-Wärmeübertrager zwischen den Thermoblechen durchströmt.

17. Reaktor nach einem der vorherigen Ansprüche, **dadurch gekennzeichnet, dass** die Reaktionsvorrichtung einer Reaktorzelle eine Mehrzahl von Kompartimenten (18, 19) aufweist, wobei ein erstes Kompartiment (18) nicht mit Katalysator befüllt ist und so konfiguriert ist, dass es von unten nach oben vom Einsatzgasgemisch oder Restgasgemisch durchströmbar ist, und wobei zum ersten Kompartiment stromabwärts angeordnete Kompartimente (19) mit Katalysator befüllt sind und so konfiguriert sind, dass sie von oben nach unten vom Einsatzgasgemisch oder Restgasgemisch durchströmbar sind.

18. Verwendung des Reaktors nach einem der vorherigen Ansprüche zur Herstellung von Methanol aus Synthesegas, wobei das Synthesegas Kohlenstoffoxide (CO, CO₂) und Wasserstoff (H₂) aufweist.

19. Verwendung nach Anspruch 18, wobei das Synthesegas kein Kohlenmonoxid oder kein Kohlendioxid aufweist.
